# FASCICULE DE BREVET EUROPEEN

(11) **EP 1 319 063 B1**
(45) Date de publication et mention de la délivrance du brevet: **18.07.2007**
(21) Numéro de dépôt: 01972175.2
(22) Date de dépôt: 20.09.2001
(51) Int. Cl.: C12N 5/06, C12N 1/04, C12P 21/00, C12Q 1/02

(54) **PROCEDE DE PROPAGATION, IN VITRO, DE L'AGENT RESPONSABLE DES ENCEPHALOPATHIES SPONGIFORMES TRANSMISSIBLES**
VERFAHREN ZUR IN VITRO VERMEHRUNG VON ERREGERN ÜBERTRAGBARER SPONGIFORMER ENCEPHALOPATHIEN
METHOD FOR IN VITRO PROPAGATION OF THE AGENT RESPONSIBLE FOR TRANSMISSIBLE SPONGIFORM ENCEPHALOPATHIES

(30) Priorité: 20.09.2000 FR 0011989
(43) Date de publication de la demande: 18.06.2003
(73) Titulaire: UNIVERSITE JOSEPH FOURIER (GRENOBLE 1), 38040 Saint Martin d'Heres (FR)
(72) Inventeur: BLANQUET GROSSARD, Françoise, F-38140 Izeaux (FR); CESBRON, Jean-Yves, F-38240 Meylan (FR); LEMAIRE VIEILLE, Catherine, F-38410 St Martin d'Uriage (FR); FOLLET, Jérôme, F-59000 Lille (FR)
(74) Mandataire: Guerre, Dominique
(86) Numéro de dépôt international: PCT/FR2001/002933
(87) Numéro de publication internationale: WO 2002/024871

(56) Documents cités:
- MOSER M ET AL: "Developmental expression of the prion protein gene in glial cells." NEURON, vol. 14, no. 3, mars 1995 (1995-03), pages 509-517, XP000998684
- ROIKHEL V M ET AL: "Persistance of the scrapie agent in glial cells from rat gasserian ganglion." ACTA VIROLOGICA, vol. 31, no. 1, 1987, pages 36-42, XP000998671
- MARKOVITS P ET AL: "Effects of in vitro infection of mouse glial and neuroblastoma cells with the scrapie agent." ANNALES DE RECHERCHES V¹T¹RINAIRES, vol. 16, no. 1, 1985, pages 111-119, XP000998670

## Description

L'invention concerne un procédé de propagation, in vitro, de l'agent responsable des encéphalopathies spongiformes transmissibles (ESTs), ou prion, permettant de conserver le pouvoir infectant du prion.

Les ESTs regroupent principalement la tremblante du mouton (ou scrapie), l'encéphalopathie spongiforme bovine, la maladie de Creutzfeldt-Jakob et le Kuru chez l'homme.

Ces maladies neurodégénératives sont caractérisées par l'apparition d'une forme anormale de la protéine PrP^{c} naturellement présente à la surface de certaines cellules, et notamment des neurones.

La protéine PrP^{c} ou protéine du prion est une sialoglycoprotéine de la surface cellulaire qui joue un rôle primordial dans les encéphalopathies spongiformes transmissibles chez l'homme et l'animal. Dans ces maladies, la PrP^{c} est convertie en une forme pathogène dénommée PrP^{sc}, qui serait une isoforme anormale de PrP^{c}, qui s'est avérée être indispensable à l'infectivité et que l'on considère aujourd'hui être l'agent infectieux de ces maladies.

Le processus de transformation de la PrP^{c} en PrP^{sc} reste indéterminé et on dispose actuellement de peu d'outils pour étudier le mécanisme d'action de cet agent infectieux, notamment en vue de sa détection et de la recherche de traitements thérapeutiques pour traiter les maladies qu'il entraîne.

Selon la publication de C. Lemaire-Vieille et al., PNAS, Vol. 97, N°10 du 09/05/2000, *Epithelial and endothelial expression of the green fluorescent protein reporter gene under the control of bovine prion protein (PrP) gene regulatory sequences in transgenic mice,* les auteurs des travaux ont obtenu plusieurs lignées de souris transgéniques qui expriment le gène « reporter » d'une protéine fluorescente (gfp), sous le contrôle de séquences de régulation du gène de la protéine PrP bovine. L'exposition à un rayonnement UV de coupes histologiques issues d'organes prélevés sur ces souris, permet de localiser la production de la fluorescence, et donc d'identifier les cellules capables d'exprimer le gène PrP. Les auteurs ont ainsi observé que les sites de production étaient les cellules de Purkinje cérébelleuses, les lymphocytes, les cellules épithéliales de la région médullaire du thymus, les kératinocytes et les cellules endothéliales des capillaires sanguins des villosités intestinales.

Ces travaux ont donc permis de disposer d'un modèle d'étude à partir duquel il est alors possible de déterminer les lieux d'expression du gène du prion.

Les auteurs de la présente invention ont découvert que les cellules gliales du système nerveux et en particulier les cellules gliales du système nerveux périphérique et les cellules de Schwann, constituent un site approprié pour la propagation, in vitro, du prion.

On ne connaît à ce jour que très peu de lignées cellulaires capables de perpétuer le pouvoir infectieux de l'agent responsable des ESTs ; il s'agit de la lignée cellulaire N2a dérivée de cellules de neuroblastome de souris [Butler, J. Virol. (1988) 62, 1558-1564], de la lignée cellulaire PC12 dérivée de cellules de phéochromocytome de rat [Rubenstein, J. Gen. Virol. (1984) 65, 2191-2198],de la lignée cellulaire GT1 dérivée de cellules neuronales issues de l'hypothalamus [Schatz, J. Virol. (1997) 11, 8821-8831] et de la lignée cellulaire 1C11 issue de cellules de neuroectoderme [Mouillet-Richard, Microbes Infect. (1999) 12, 969-976].

Dans l'article V.M. Roikhel et al., Acta Virologica, vol. 31, N°1 (1987) 36-42, les auteurs ont mis en évidence que des cellules issues du ganglion de Gasser de rat, les cellules NGUK-I, pouvaient être infectées in vitro et conserver, leur pouvoir infectieux. Les cellules NGUK-I ont été caractérisées et possèdent un profil de croissance qui est celui des astrocytes et un profil morphologique qui est celui des cellules épithéliales (A.P. Avtsyn et al., Tsitologiia (janvier 1989) 1, 97-102).

Toutes les cellules précédemment citées ne sont pas des cellules gliales et la découverte selon la présente invention est surprenante en ce que les cellules gliales ne sont pas des cellules neuronales et que l'infectivité qui s'y développe, comme cela est montré ci-après, est forte.

Ainsi, un premier objet de l'invention est un procédé de propagation, in vitro, du prion, selon lequel on dispose d'une culture ou d'une lignée de cellules de Schwann et on infecte la culture ou la lignée par l'agent responsable des ESTs ou prion. Plus particulièrement, ledit agent est d'origine murine.

Avant de décrire l'invention plus en détails, certains termes employés dans la description et les revendications sont ci-après définis.

Par propagation du prion dans une culture cellulaire, on comprend selon l'invention, qu'après infection, ou infestation, d'au moins une cellule de la culture cellulaire de départ ou de la lignée cellulaire de départ, on conserve le pouvoir infectieux du prion dans les cellules dérivées, c'est-à-dire les cellules résultant de sous-cultures.

Le prion utilisé pour infecter la culture ou lignée de départ est choisi parmi les souches de prion telles que la souche Chandler ou la souche RML (Rocky Mountain Laboratory), ou parmi les cellules d'une culture primaire, c'est-à-dire des cellules directement prélevées à partir d'un tissu infecté. A titre d'exemple, il peut s'agir de tissus cérébraux prélevés post-mortem sur des souris.

Des variantes préférentielles du procédé ci-dessus et d'autres objets de l'invention sont maintenant présentés.

Le procédé est avantageusement mis en oeuvre à partir d'une culture ou d'une lignée de cellules de Schwann d'origine murine ; une lignée préférée est la lignée MSC-80.

L'invention concerne aussi une lignée de cellules de Schwann, infectées par le prion, capable de propager le prion ; les cellules de la lignée sont avantageusement dérivées de cellules de la lignée de cellules de Schwann murine, MSC-80, après infection par le prion.

Un autre objet de l'invention est un procédé d'évaluation de l'efficacité d'une molécule, d'un agent ou d'une composition, pour diminuer ou inhiber l'infectiosité du prion, procédé selon lequel on met en contact une culture de cellules de Schwann infectées par le prion ou une lignée cellulaire de l'invention, ou un extrait de celles-ci, avec ladite molécule, ledit agent ou ladite composition, à des doses prédéterminées, et on détecte la diminution ou l'inhibition de l'infectiosité du prion. L'étape de détection peut être réalisée chez des souris Tga 20.

L'invention apporte aussi un procédé pour la détection, et éventuellement la quantification du prion, dans un échantillon biologique, selon lequel on met ledit échantillon en contact avec une culture ou une lignée de cellules de Schwann, dans des conditions permettant l'infection de cette dernière, et on détecte l'infection ou la non infection de ladite lignée par ledit échantillon. De préférence, la lignée de cellules de Schwann est la lignée MSC-80.

Encore un autre objet de l'invention est l'utilisation de la lignée cellulaire MSC-80 pour propager, in vitro, le prion.

La présente invention est ci-après illustrée par les exemples suivants.

### Exemple 1 : infection de la lignée MSC-80 et mise en évidence de cette infection

Des cellules de Schwann murines MSC-80 sont cultivées en boîte de six puits (2x10⁵ cellules par puits), 48 heures avant l'infection, dans un milieu de culture consistant en du milieu de Eagle modifié par Dulbecco (DMEM) complété avec 10% de sérum de veau foetal (Life Technologies, Paisley, UK), de la L-glutamine 2 mM final (Life Technologies) et 100 U/ml de pénicilline /100µg de streptomycine (SEROMED).

Un homogénat à 10% (poids / volume) de cerveaux de souris infectés par la souche de prion RML (Rocky Mountain Laboratory) est obtenu par broyage mécanique dans le milieu de culture précédemment décrit, suivi d'un passage à travers des aiguilles de 16, puis 22 gauges, et chauffé à 80°C pendant 20 minutes.

Les cellules MSC-80 sont ensuite mises en contact avec 1 ml par puits d'homogénat dilué à 2%, à 37°C pendant 72 heures. L'inoculum et le milieu ainsi utilisés sont renouvelés toutes les 24 heures. Après 72 heures, le surnageant du milieu de culture est remplacé par 2 ml de milieu de culture frais. Les cellules sont cultivées jusqu'à confluence. Le contenu de chaque puits est alors réparti dans des fiasques de culture de 75 cm², puis de 150 cm², et est repiqué au 1/10^{ème} dans des flasques de 150 cm² tous les 7 jours.

La présence de la protéine PrP^{sc} est détectée par Western-Blot. Les cellules (8x10⁶) sont lysées dans 200 µl de tampon (Triton-DOC) pendant 1 5 minutes sur de la glace. La quantité de protéines du surnageant est dosée et ajustée à 1 mg / ml avec du tampon de lyse.

Un milligramme de protéines totales est alors digéré par 20 µg de protéinase K (BOEHRINGER MANNHEIM, Meylan, France) à 37°C pendant 30 minutes, de manière à détruire les protéines PrP^{c} (normales). La réaction est arrêtée pendant 5 minutes sur la glace, après l'ajout à 2 mM final de fluorure de phényl-méthyl-sulfonyle (SIGMA, St Louis, MO). Le produit de digestion est centrifugé pendant 45 minutes à 14 000g. Le culot est repris dans 20 µl de tampon de Laemmli, chauffé 5 minutes à 100°C, et déposé sur un gel de polyacrylamide 12%.

Après la migration dans un tampon Tris-Glycine à 50 mA pendant 2 heures, le gel est électrotransféré sur une membrane de nitrocellulose pendant 2 heures. La protéine PrP^{sc} est alors détectée en utilisant un mélange à équivalence de trois anticorps monoclonaux (SAF60, SAF69 et SAF70 ; GRASSI, CEA Saclay, France). La présence de ces anticorps est révélée par un anticorps anti-immunoglobuline de souris couplé à la peroxydase qui permettra la révélation par chémiluminescence (Kit ECL, AMERSHAM LIFE SCIENCE, Little Chalfont, UK).

### Exemple 2 : Mise en évidence de la propagation du prion

Le pouvoir infectieux du prion propagé selon l'Exemple 1 a été analysé par inoculation intracérébrale de souris Tga20, disponibles à l'Institut de Neuropathologie de Zürich (S. Brander et al., Nature (1996) 379, 339-343 ; M. Fisher et al., EMBO J., (1996) 15, 1255-1264).

Un premier lot de souris Tga20 a été inoculé avec la souche de prion de Chandler, un second lot de souris a été inoculé par des cellules de Schwann murines témoin MSC^{Ct}, et un troisième lot de souris a été inoculé avec des cellules de Schwann infectées MSC^{Ch}, décrites à l'Exemple 1.

Ce troisième lot a été obtenu par inoculation des souris par des extraits de cellules infectées de l'Exemple 1, résultant du septième passage.

Au dernier stade de la maladie, la PrP^{sc} est identifiée par Western-Blot après digestion partielle avec de la protéinase K (cf. exemple 1). Le profil obtenu dans les extraits de cerveaux des souris inoculées avec les cellules MSC^{Ch} est identique à celui obtenu chez les souris inoculées avec la souche Chandler.

L'analyse immunohistochimique avec des anticorps dirigés contre la protéine gliale fibrillaire acide (GFAP) révèle, dans le tégument du mésencéphale des souris inoculées avec les cellules MSC^{Ch}, une hyperplasie et une hypertrophie astrocytaire. Ces cerveaux présentent aussi une vacuolisation du neurophile visible après coloration à l'hématoxiline-éosine.

88 jours après l'inoculation, les souris infectées précitées présentent une hyperactivation et une perte de l'équilibre. Elles meurent au bout de 91,5 ± 5 jours.

Les mêmes observations ont été observées en remplaçant ce troisième lot, par un quatrième lot obtenu par inoculation des souris par des extraits de cellules infectées de l'Exemple 1, résultant du vingt-deuxième passage.

## Revendications

1. Procédé de propagation, in vitro, de l'agent responsable des encéphalopathies spongiformes transmissibles (ESTs), ou prion, **caractérisé en ce qu'**on dispose d'une culture ou d'une lignée de cellules gliales du système nerveux périphérique choisies parmi les cellule de Schwann, et **en ce qu'**on infecte ladite culture ou lignée par l'agent responsable des encéphalopathies spongiformes transmissibles (ESTs) ou prion.

2. Procédé selon la revendication 1, **caractérise en ce que** les cellules de Schwann de ladite culture ou lignée sont d'origine murine.

3. Procédé selon la revendication 2, **caractérisé en ce que** la lignée est la lignée MSC-80.

4. Procédé selon l'une quelconque des revendications 1 à 3, **caractérisé en ce qu'**on infecte ladite culture ou ladite lignée par l'agent scrapie murin.

5. Lignée de cellules de Schwann infectées par le prion, capable de propager le prion.

6. Lignée selon la revendication 5, **caractérisée en ce que** les cellules de la lignée sont dérivées de cellules de la lignée de cellules de Schwann murine, MSC-80, après infection par le prion.

7. Procédé d'évaluation de l'efficacité d'une molécule, d'un agent ou d'une composition pour diminuer ou inhiber l'infectiosité du prion, **caractérisé en ce qu'**on met en contact une culture de cellules de Schwann infectées par le prion ou une lignée cellulaire selon la revendication 5 ou 6, ou un extrait de celles-ci, avec ladite molécule, ledit agent ou ladite composition, à des doses prédéterminées, et on détecte la diminution ou l'inhibition de l'infectiosité du prion.

8. Procédé selon la revendication 7, **caractérisé en ce qu'**on détecte la diminution ou l'inhibition de l'infectiosité du prion chez des souris Tga 20.

9. Procédé pour la détection, et éventuellement la quantification du prion, dans un échantillon biologique, **caractérisé en ce qu'**on met ledit échantillon en contact avec une culture de cellules de Schwann ou une lignée de cellules gliales selon la revendication 5 ou 6, dans des conditions permettant l'infection de cette dernière, et **en ce qu'**on détecte l'infection ou la non infection de ladite lignée par ledit échantillon.

10. Procédé selon la revendication 9, **caractérisé en ce que** la lignée est la lignée MSC-80.

11. Utilisation de la lignée cellulaire MSC-80 pour propager, in vitro, le prion.

## Claims

1. A method for in vitro propagation of the agent responsible for transmissible spongiform encephalopathies (TSEs), or prion, **characterized in that** a glial cell culture or a glial cell line of the peripheral nervous system chosen from Schwann cells, is provided and **in that** said culture or line is infected with the agent responsible for transmissible spongiform encephalopathies (TSEs), or prion.

2. The method as claimed in claim 1, **characterized in that** the Schwann cells of said culture or line are of murine origin.

3. The method as claimed in claim 2, **characterized in that** the line is the MSC-80 line.

4. The method as claimed in either one of claims 1 to 3, **characterized in that** said culture or said line is infected with the murine scrapie agent.

5. A line of Schwann cells infected with the prion, capable of propagating the prion.

6. The line as claimed in claim 5, **characterized in that** the cells of the line are derived from cells of the murine Schwann cell line MSC-80, after infection with the prion.

7. A method of evaluating the effectiveness of a molecule, of an agent or of a composition in decreasing or inhibiting the infectiousness of the prion, **characterized in that** a culture of Schwann cells infected with the prion or a cell line as claimed in claim 5 or 6, or an extract thereof, is brought into contact with said molecule, said agent or said composition, at predetermined doses, and the decrease in or the inhibition of the infectiousness of the prion is detected.

8. The method as claimed in claim 7, **characterized in that** the decrease in or the inhibition of the infectiousness of the prion is detected in Tga 20 mice.

9. A method for detecting and, optionally, quantifying the prion, in a biological sample, **characterized in that** said sample is brought into contact with a culture of Schwann cells or a line of glial cells as claimed in claim 5 or 6, under conditions which allow the infection thereof, and **in that** the infection or noninfection of said line with said sample is detected.

10. The method as claimed in claim 9, **characterized in that** the line is the MSC-80 line.

11. The use of the MSC-80 cell line for propagating the prion in vitro.

## Patentansprüche

1. Verfahren zur In-vitro-Vermehrung des Erregers, der für übertragbare spongiforme Enzephalopathien (EST) verantwortlich ist, d.h. von Prion, **dadurch gekennzeichnet, dass** man über eine Kultur oder eine Linie von Gliazellen des peripheren Nervensystems, die aus Schwann-Zellen ausgewählt sind, verfügt, und **dadurch**, dass man die Kultur oder Linie mit dem Erreger, der für übertragbare spongiforme Enzephalopathien (EST) verantwortlich ist, d.h. mit Prion, infiziert.

2. Verfahren nach Anspruch 1, **dadurch gekennzeichnet, dass** die Schwann-Zellen der Kultur oder der Linie murinen Ursprungs sind.

3. Verfahren nach Anspruch 2, **dadurch gekennzeichnet, dass** es sich bei der Linie um die Linie MSC-80 handelt.

4. Verfahren nach einem der Ansprüche 1 bis 3, **dadurch gekennzeichnet, dass** man die Kultur oder die Linie mit dem Mäuse-Scrapie-Erreger infiziert.

5. Schwann-Zelllinie, die mit Prion infiziert ist und in der Lage ist, Prion zu vermehren.

6. Linie nach Anspruch 5, **dadurch gekennzeichnet, dass** die Zellen der Linie von den Zellen der murinen Schwann-Zelllinie, MSC-80, nach Infektion mit Prion stammen.

7. Verfahren zur Untersuchung der Wirksamkeit eines Moleküls, eines Mittels oder einer Zusammensetzung zur Verringerung oder Hemmung der Infektiosität von Prion, **dadurch gekennzeichnet, dass** man eine Kultur von Schwann-Zellen, die mit Prion infiziert sind, oder eine Zelllinie nach Anspruch 5 oder 6 oder einen Extrakt von diesen mit dem Molekül, dem Mittel oder der Zusammensetzung in zuvor bestimmten Dosen in Kontakt bringt und die Verringerung oder Hemmung der Infektiosität von Prion ermittelt.

8. Verfahren nach Anspruch 7, **dadurch gekennzeichnet, dass** man die Verringerung oder Hemmung der Infektiosität von Prion bei Tga-20-Mäusen ermittelt.

9. Verfahren zur Ermittlung und gegebenenfalls zur Quantifizierung von Prion in einer biologischen Probe, **dadurch gekennzeichnet, dass** man die Probe mit einer Kultur von Schwann-Zellen oder einer Linie von Gliazellen nach Anspruch 5 oder 6 unter Bedingungen in Kontakt bringt, die deren Infektion gestatten, und **dadurch**, dass man die Infektion oder Nicht-Infektion dieser Linie durch die Probe ermittelt.

10. Verfahren nach Anspruch 9, **dadurch gekennzeichnet, dass** es sich bei der Linie um die MSC-80-Linie handelt.

11. Verwendung der MSC-80-Zelllinie zur In-vitro-Vermehrung von Prion.
